# EUROPEAN PATENT SPECIFICATION

(11) **EP 3 427 789 B1**
(45) Date of publication and mention of the grant of the patent: **05.07.2023**
(21) Application number: 17180658.1
(22) Date of filing: 11.07.2017
(51) Int. Cl.: A61N 1/05, A61N 1/362, A61N 1/20

(54) **IMPLANTABLE DIRECT-CURRENT ELECTRODE ASSEMBLY**
IMPLANTIERBARE GLEICHSTROM-ELEKTRODENANORDNUNG
ENSEMBLE D'ÉLECTRODES À COURANT CONTINU IMPLANTABLES

(43) Date of publication of application: 16.01.2019
(73) Proprietor: Berlin Heals GmbH, 10719 Berlin (DE)
(72) Inventor: MÜLLER, Johannes, 10717 Berlin (DE)
(74) Representative: Liebetanz, Michael

(56) References cited:
- WO-A2-2017/021255
- US-A1- 2004 186 546
- US-A1- 2004 230 274
- US-A1- 2008 195 161
- US-A1- 2010 152 812
- US-A1- 2017 001 001

## Description

### TECHNICAL FIELD

The present invention relates to an implantable direct-current electrode assembly with two implantable electrodes and a control unit, to which the first and the second electrodes are electrically connected through leads. The control unit is designed to establish a potential difference between the two electrodes, so that a direct current can flow between the two electrodes.

### PRIOR ART

Such an electrode assembly, according to the preamble of the claim 1 is known from WO 2017/021255, wherein the first coil electrode is positioned between the tricuspid valve and the apex of the right ventricle lying opposite the tricuspid valve and the pulmonary valve touching the right ventricular wall. A second electrode is positioned on the epicardial site of the left ventricular wall (touching the left ventricular wall externally) or such a second electrode is positioned inside the coronary sinus pushed downwards to the left ventricle apex of the left ventricle. Then a direct-current flow is initiated between the coil electrode in the right ventricle and the coil electrode in the coronary sinus (sinus coronarius) which leads to an electric current flow through the left ventricle wall across the septum. It is furthermore known from WO 2006/106132 or WO 2017/021255 that a damaged heart muscle can be treated for prolonged time periods through application of a direct-current which strength is far below the threshold which is sufficient to induce a contraction of the heart. The current was defined as a direct-current that could not excite the cardiomyocytes or introduce a contraction of the heart.

### SUMMARY OF THE INVENTION

Based on this prior art it is an object of the present invention to prevent or treat already existing atrial fibrillation.

This object is achieved for an implantable direct-current electrode assembly with the features of claim 1.

It has been found by the inventors, that providing an electrode assembly with a shorter first electrode to be placed inside the right atrium attached to the atrium wall and a second electrode in the coronary sinus with a shorter introduction portion into the sinus, while providing a direct-current flow between the two atrium portions suppresses the initiation of atrial fibrillation or diminish or eliminate an already existing atrial fibrillation. The shorter introduction portion makes the second conducting electrode part to be positioned horizontally just above the valve of the left atrium.

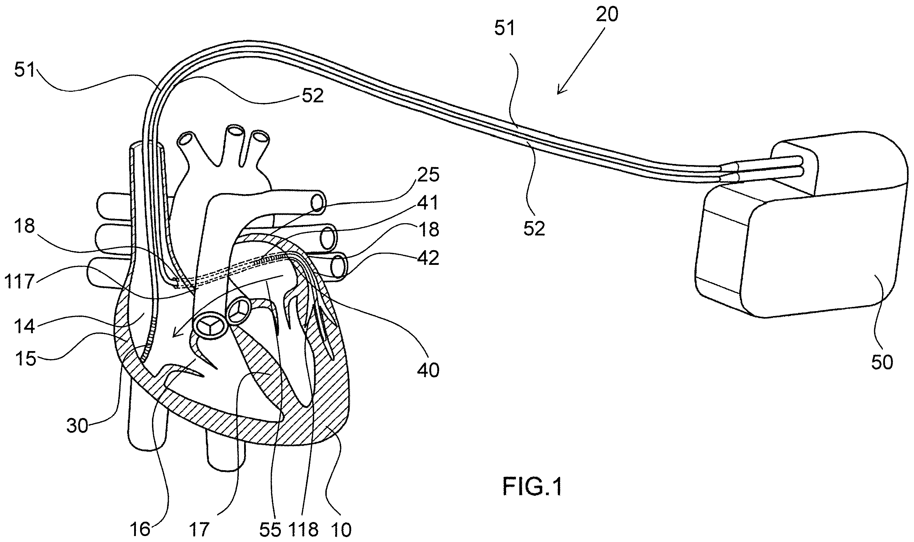

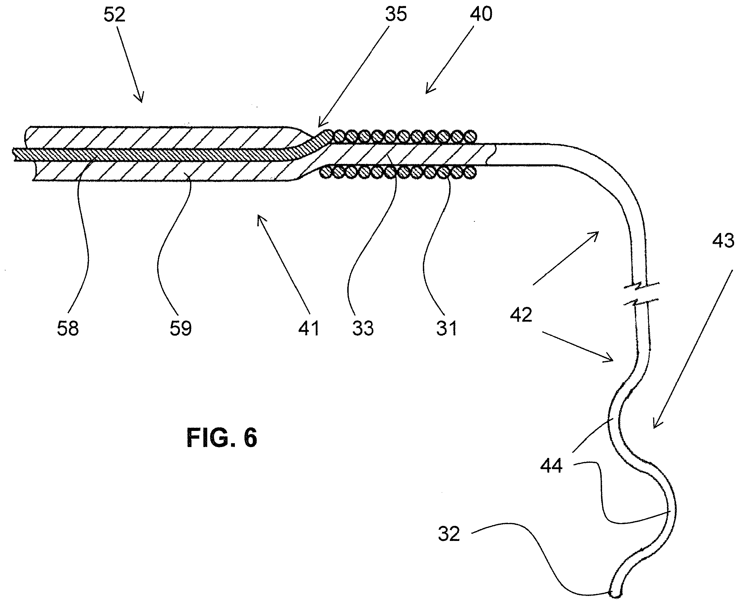

It comprises an isolated anchoring portion extending beyond the electrode portion ending in a tip having a predetermined length. Said predetermined length of the isolated anchoring portion allows the electrode to follow the bend of the vena cardiaca magna into the direction of the left ventricle. Preferably, the isolated anchoring portion comprises pre-bent curved structure to be positioned against a plurality of contact points inside the cross-section of the tapering vena cardiaca magna until the tip of the isolated anchoring portion. In a simpler embodiment, the cross-section of the tapering vena is filled by the tip of the isolated anchoring portion as shown in Fig. 1, but the version of the tip portion Fig 6 is preferred by far. The isolating portion can have the same diameter as the core around which the helix-shaped electrode is wound. It can have a constant diameter until the rounded tip for a specific predetermined fixation point, maintaining the electrode portion in the region before the bend of the vena.

The electric connection between the electrically connected first and second electrodes and the control unit can be single wired isolated lines.

The first or second electrode can comprise solely one or more spirals wound around an isolating core being in one piece with the isolating cladding of the electric connection with no or one or more attachment means, but comprising the naked anchoring portion attached in one piece.

On the other side attachments means can be are provided at the first electrode comprising one or more free ends. There can also be provided three or four free ends spanning a triangle or square, respectively, in a plane perpendicular to the longitudinal direction of the electrode in front of the electrode end.

There can be provided two or three groups of one or two free ends extending essentially transverse to the longitudinal direction of the electrode, each group provided between a transition portion at the beginning of the electrode and the tip of the electrode, preferably at the beginning, the tip and in the case of three groups in the middle between the beginning and the end of the electrode. Each said group can comprise two free ends having an angle between 45 and 90 degrees in a plane perpendicular to the longitudinal direction of the electrode in front of the electrode end.

These free end(s) can be tapering each into an anchoring spike or into an anchoring hook.

The coil electrode configured as a heart-external coil electrode to be attached to the exterior wall of the left atrium can also be a patch electrode.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a perspective schematic view of a heart and an electrode assembly according to an embodiment of the invention,
- Fig. 2: shows a perspective schematic view of a heart and an electrode assembly according to an example not according to the invention,
- Fig. 3: shows the distal end of a coil electrode for the first electrode according to an embodiment of the invention,
- Fig. 4: shows a coil electrode to be attached at the outside of the atrium wall or to be placed in the coronary sinus;
- Fig. 5: shows a further embodiment for the second outer atrial electrode; and
- Fig. 6: shows a further embodiment for the second inner atrial electrode used in connection with the assembly according to Fig. 1.

### DESCRIPTION OF PREFERRED EMBODIMENTS

Fig. 1 shows a perspective schematic view of a heart 10 and an electrode assembly 20 according to a first embodiment of the invention.

The implantable electrode assembly 20 comprises two leads having two implantable electrodes 30 and 40, as well as an electronic control unit 50, usually provided in a case, provided in a distance from the heart 10, wherein also a battery is placed in the case to provide the necessary power supply.

The two electrodes 30 and 40 are connected via two single wire connections 51 and 52 with said electronic control unit 50. The two single wire connections 51 and 52 forming the flexible leads as well as the control unit case 50 are electrically isolated against the environment. The control unit 50 is configured to create a potential difference between the two electrodes 30 and 40 extending beyond the two isolated single wire connections 51 and 52, which potential difference allowing a direct-current to flow between the two electrodes 30 and 40 along arrow 55.

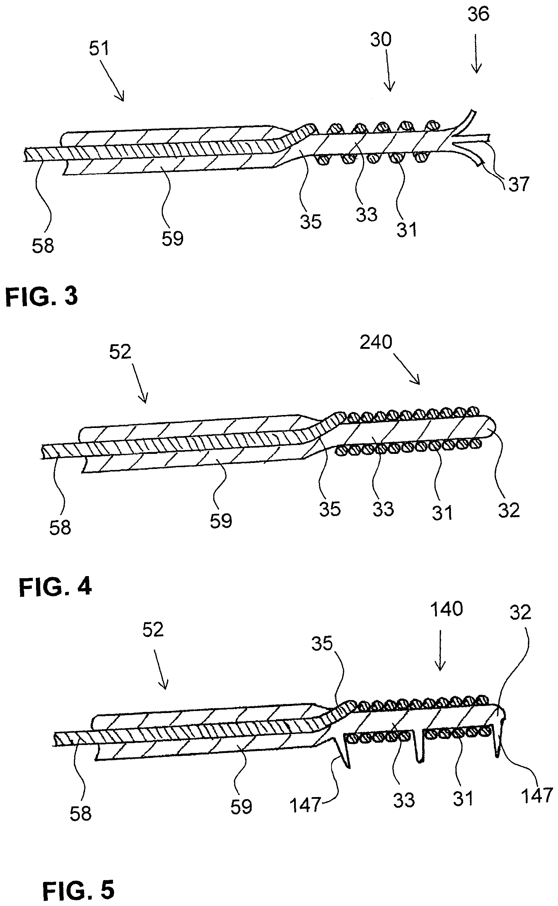

The first electrode 30 is a right atrium electrode, configured to be positioned in the right atrium and is a coil electrode. The length of the coil atrial electrode 30, i.e. the non-isolated part of the lead reaching from the electronic control unit 50 to the transition portion 35, is predetermined through the distance between the entry of the right atrium 14 and the tricuspid valve 16, especially chosen between 6 and 8 cm and is provided with an anchoring tip 37 (not represented in Fig. 1 but shown in Fig. 3) to be positioned preferably within the right atrium touching the atrial wall 15 from the inside. There are other attachment possibilities as noted in connection with the second electrode (in the embodiment according to electrode 140). The total length of the lead plus electrode can be between 50 and 80 cm.

The second electrode 40 is a coronary sinus electrode, configured to be positioned in the coronary sinus 18 and is also a coil electrode. The coronary sinus coil electrode 40 comprises a smaller diameter than the coil atrial electrode 30 since it has to enter the coronary sinus 18 and has to push forward into the vena cardiac magna until the vena cardiac magna bends into the direction of the left ventricle. The coil electrode 40 is attached at a shorter isolated introduction portion 41 so that it is not pushed into the tapered end portion of the coronary sinus, but is positioned at the height of the left atrium 24 near the left atrium wall 25. The coil electrode 40 further comprises a prolongation as an isolating anchoring portion 42 as shown in connection with the embodiment of Fig. 6. The length of the anchoring portion 42 can be between 3 and 6 times the length of the electrode portion 40. In other words, the lead element comprises an isolated wire portion 52 with an introduction portion 41, wherein the electrode portion 40 follows, wherein a further isolation core part 42 is provided extending beyond the electrode portion 40 until the tip 33, wherein the length of this isolation core part 42 is predetermined to allow to be lodged from the point in the vena cardiaca magna where the vena cardiaca magna bends into the direction of the left ventricle. Fig. 1 shows a more or less straight end portion of the isolation core part 42. It is, however, preferred that the isolation core part 42 does not fill the volume of the lumen of the size reducing vena cardiaca magna but that the portion until the tip 32 is bent in several preformed waves to push against opposite side of the lumen of the vena cardiaca magna to position the second electrode 40 at a predetermined place and securely lodged there.

When a potential difference is applied between the two electrodes 30 and 40 by means of the electronic control unit 50, since the wires 51 and 52 are isolated against the environment, a direct-current is flowing according to arrow 55 through the heart muscle in the biatrial area, i.e. across from the left atrium 14 to the right atrium 24 through the atrial septum 117. Depending on the preferred direction of the current flow, the electrode 30 can be set as a cathode or anode with the electrode 40 as matched counter electrode accordingly.

The electronic control unit 50 is preferably programmable to predetermine a time interval within which the potential difference is maintained to obtain the direct-current flow, which can range from some minutes, over an interval of 30 minutes or an hour until a number of hours, days or months, wherein the electrode 30 is the cathode to define the current flow. After a predefined time, the current direction can be inverted, wherein the electrode 40 becomes the cathode and a similar time interval is provided after such a first time interval. This changes the direction of current flow according to arrow 55. This sequence of change of current flow inversion can be continued for prolongated periods of time, e.g. for up to several months or even years.

It is also possible to change the current strength while inverting the current flow, since the impedance between the two electrodes 30 and 40 can be dependent on the direction 55 of the current flow. The amount of the direct current flow is predetermined to be far below the stimulation threshold, especially chosen to have a current density of 0.1 microampère/cm² to 1 milliampère/cm². The electronic control unit 50 can comprise a control to maintain the current density below a maximum threshold.

Inverting a current flow has to be executed quasi-stationary, i.e. decreasing the current density over several minutes to zero and raising it with the opposite leading sign to the predetermined new direct current density level to avoid any rhythm disturbances which can potentially lead to dys- or arrhythmia.

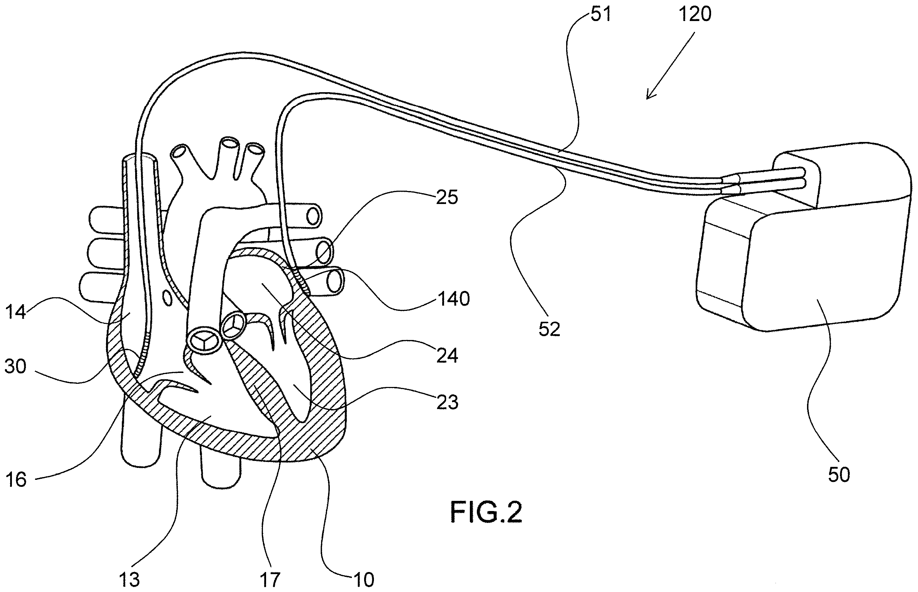

Fig. 2 shows a perspective schematic view of a heart 10 and an electrode assembly 120 according to an example not according to the invention. According to this embodiment, a first electrode 30 is provided in the same way as in the first embodiment in the wall 15 of the right atrium 14. The second electrode 140 is attached at the outer wall 25 of the left atrium 15. This can be done through stitching of the electrode 140, positioned just in parallel and along on the left atrium wall 25 (stitching not actually shown in Fig. 2) or the electrode 140 can comprise two or more lateral spikes 147, as shown in Fig. 5, which are entering the left atrium wall 25. An additional planar attachment as covering the electrode via a patch is possible as well. Such a patch can be a mesh or a thin electro-conductive film covered on the backside with silicone.

It is also possible to use a patch electrode at the place of a coil electrode 140. Then a patch electrode as disclosed in WO 2016/016438 or in WO 2006/10132 can be used with the proviso that it is attached, especially stitched at the epicardium of the left atrium or at the pericardium covering the left atrium. Such a patch electrode has the advantage of a larger surface reducing the current density crossing the heart portions and at the same time allowing to cover a larger portion of the surface atrial septum 117, when the direct current flow according to arrow 55 extends between the smaller rectangle (in a cross-section approach) of the coil electrode 30 and the entire surface of a patch attached near the left atrial external wall.

Fig. 2 has less reference numerals than Fig. 1, so that additional references are introduced here, which also applies to Fig. 1. The heart 10 is shown with the right ventricle 13 and the left ventricle 23, separated by the septum 17. The right atrium 14 is separated by valve 16 from the right ventricle 13. The outer wall of the right atrium 14 is right atrium wall 15. On the other side, the left atrium 24 has the left atrium wall 25 within which is located portions of the coronary sinus 18 within which is provided the second electrode 40 of the first embodiment.

Fig. 3 shows the distal end of a coil electrode 30 for the first electrode according to an embodiment of the invention, i.e. to be applied for an embodiment of the assembly according to Fig. 1 or Fig. 2. The electrode is far simpler constructed than usual coil electrodes for pacemakers etc.. The single-wire 51 comprises an electrically conductive core 58 and an isolating cladding 59. The cladding 59 ends in an area in front of the electrode 30 comprising the helix shaped distal end having the electrically conductive coil 31 around the electrode core 33. The transition portion 35 comprises the spot where the single wire electrically conductive core exits the cladding 59 which continues as electrode core 33. The diameter of the core 33 is less than the diameter of the cladding 59 as well as that the helix shaped electrically conductive coil 31 has a smaller diameter than the cladding 59 or has at the most its diameter.

The coil electrode 30 of Fig. 3 comprises a tip 36 with three free ends 37 building an anchoring tip, especially a tip spanning a triangle at the three tips. There are two principle methods of fixation of the electrode to differentiate. One is a traumatic one the other one an atraumatic fixation. Various available anchoring facilities for the electrodes consists of flanges, books, prongs, jaws and various types of screw and spiral tips. Preferably, the ends are provided in a same plane which is perpendicular to the longitudinal axis of the electrode tip. The anchoring tip is connected with the core 33 and then with the cladding 59 and are made of the same isolating material. This allows to anchor the electrode tip 36 at the right atrium wall 15 from the inside without providing any or only little current or potential to the atrium wall 15. The direct current is traversing the upper portion of the septum at the level between the right atrium 14 and the left atrium 24. If there is a small direct current flowing through the right atrium wall 15 this could prevent atrial fibrillation starting from the right atrium as such.

Fig. 4 shows a coil electrode 240 to be attached at the outside of the atrium wall as exterior electrode 140 as shown in the embodiment of Fig. 2 or to be placed in the coronary sinus 18 according to the embodiment of Fig. 1 (however, with tip 32 directly ending in front of the electrode portion 40). Similar features have received the same or similar reference numerals throughout the description. The non-conducting portion of the electrode 40 is similar to the electrode 30. One difference is inter alia that the helix shaped conductive part 31 is closer packed than in Fig. 3. However, it is also possible to use the closer packed part 31 in Fig. 3 and the lesser packed conductive portion of Fig. 3 in the embodiment of Fig. 4. The closed packed portion 31 provides a full conductive surface in front of the septum 17.

The main difference between the two electrodes is the blunt end 32 allowing to push the electrode 40 into the coronary sinus 18, wherein also here the electric conductive part has a smaller or at most similar diameter than the cladding 59 of the isolated part of the introduction portion 41. The electrode 40 is positioned in the coronary sinus 18 preferably in a way that the electrode is similar to parallel to the heart valves or parallel to the first electrode 30 or in between these two positions.

It is also possible to use this electrode 40 as second electrode in the embodiment of Fig. 2. Then the electrode is stitched at the outer surface of the left atrium wall 25 and can additionally be covered by a patch also attached to the atrium wall 25, especially with single stitches or sutures. Use of glue is possible but reduces the conductivity towards the electrode.

Fig. 5 shows a further embodiment for the outer atrium electrode 140. The main difference between the embodiment of Fig. 4 and the embodiment of Fig. 5 lies in the side spikes 147. There are provided three or twice time three in an angle of between 45 and 90 degrees on one side of the tip portion seen in a cross section view. The spikes 147 are provided at the transition portion 35, at the rounded tip 32 as well as in the middle between these two length positions.

Fig. 6 shows a further embodiment for the second inner atrial electrode 40 as used in connection with the embodiment of the assembly according to Fig. 1. The second electrode 40 is a coronary sinus electrode, configured to be positioned in the coronary sinus 18. The initial introduction portion 52 as well as the electrode portion 40 beyond the transition portion 35 is shown as positioned in the coronary sinus 18 and in the vena cardiac magna until the vena cardiac magna bends into the direction of the left ventricle. Beyond the coil electrode 40 is attached an isolated anchoring portion 42 which follows the bending of the vena cardiac magna and is pushed into the tapered end portion of the coronary sinus.

Since the isolated anchoring portion 42 is usually several times (3 to 6 times) longer than the electrode portion 41, the end until the tip 32 is shown separately in Fig. 6. It is preferred that the diameter of the end of the isolated anchoring portion 42 is e.g. smaller and comprises a number of preformed bends in several preformed waves to push against opposite side of the lumen of the vena cardiaca magna to position the second electrode 40 at a predetermined place and securely lodged there. Then the isolated anchoring portion 42 is not inferring with the blood flow in the vessel.

The length of this isolation core part 42 is predetermined to allow to be lodged from the point in the vena cardiac magna where the vena cardiac magna bends into the direction of the left ventricle. Preferably, when advancing the electrode 30, a mandarin is lodged in a hollow preformed tip portion 43 to straighten the preformed tip portion 43. When this preformed tip portion 43 is in position beyond the bent, then the mandarin is retracted and the (e.g. memory form) metal bends again into its original shape, preferable providing a plurality of contact points 44 against opposite walls of the vena cardiaca magna. It is also possible that the preform bends back into a helix-shaped form positioned like a stent in the vessel; then the preformed tip portion 43 does not have distinct contact points but is pushed in a helix shape from the inside against the vessel walls..

Thus, the second electrode 40 is also positioned at a predetermined place and securely lodged there with contact points 44. The diameter of the isolation core part 42 can therefore be constant and just end in a rounded tip 32. Fig. 6 shows that it is a flexible part 42, wherein the isolated anchoring portion 43 comprises pre-bent curved structure to be positioned against a plurality of contact points 44 inside the cross-section of the tapering vena cardiaca magna until the tip 32 of the isolated anchoring portion 42. Other e.g. memory metal applications are possible to provide a good anchoring in the vena cardiaca magna.

The present invention allows a biatrial monophasic electrical sub-stimulation. The direct-current flow remains largely below-sub-threshold levels, i.e. without activating the physiologic conduction system. The first electrode 30 is positioned in the right atrial appendage 14 and the other electrode 40 or 140 is positioned over the left atrium 24 either at the left posterior atrial wall 15 or within a coronary sinus. In the letter case, the introduction of the electrodes 30 and 40 into the patients could be accomplished by intravenous access, otherwise a transthoracic assess would be necessary. The longstanding provision of sub-threshold direct-current reverses the pathological structural remodelling of atria 14 and 24 in patients with persistent atrial fibrillation or tachyarrhythmias.

The electric current enhances the proliferation of cardiomyocytes and modulates the expression of metalloproteinases and their inhibitors. Furthermore, the direct-current stimulation modulates the expression of pro-inflammatory cytokines.

### LIST OF REFERENCE SIGNS

| | | | |
|---|---|---|---|
| 10 | heart | 38 | core |
| 12 | right half of the heart | 39 | transfer area |
| 13 | right ventricle | 40 | coil coronary sinus electrode |
| 14 | right atrium | 41 | introduction portion |
| 15 | right exterior atrium wall | 42 | anchoring portion |
| 16 | tricuspid valve | 43 | preformed tip portion |
| 17 | septum | 44 | contact point |
| 18 | coronary sinus | 50 | control unit |
| 20 | electrode arrangement | 51 | single-wire connection |
| 22 | left half of the heart | 52 | single wire connection |
| 23 | left ventricle | 55 | direction of current flow |
| 24 | left atrium | 58 | single wire connection |
| 25 | left exterior atrium wall; epicardium of the left atrium | 59 | cladding |
| | | 117 | atrial septum |
| 30 | coil atrial electrode | 118 | vena cardiac magna turned to the left ventricle |
| 31 | electrically conductive coil | | |
| 32 | blunt end | 120 | electrode arrangement |
| 33 | electrode core | 140 | coil external atrium electrode |
| 35 | transition portion | 147 | side spike |
| 36 | electrode tip | 240 | coil coronary sinus electrode |
| 37 | anchoring tip | | |

## Claims

1. An implantable direct-current electrode assembly (20) with two implantable electrodes (30, 40) and a control unit (50), to which the first (30) and the second (40) electrodes are electrically connected through leads, wherein the control unit (50) is configured to establish a potential difference between the two electrodes (30, 40), so that a direct current can flow (55) between the two electrodes (30, 40), wherein the first electrode (30) is a coil electrode configured to be provided in the right half (12) of the heart (10) and has a predetermined length, and wherein the second electrode is a counter-electrode (40), wherein this counter-electrode is a coil electrode (40) having an electrode portion (31) configured to be positioned in the coronary sinus,
**characterized in that**
the coil electrode (40) is configured to be positioned in the coronary sinus at the height of the left atrium (24) and that beyond the electrode portion (31) of the coil electrode (40) is attached an isolated anchoring portion (42) extending beyond the electrode portion (31) ending in a tip (32) with a predetermined length of the isolated anchoring portion (42) to follow the bend of the vena cardiaca magna into the direction of the left ventricle (118).

2. The electrode assembly according to claim 1, wherein the isolated anchoring portion (42) comprises pre-bent curved structure (43; 44) to be positioned against a plurality of contact points (44) inside the cross-section of the tapering vena cardiaca magna until the tip (32) of the isolated anchoring portion (42).

3. The electrode assembly according to claim 1 or 2, wherein the electric connection between the electrically connected first (30) and second (40) electrodes and the control unit (50) are single wired isolated lines (51, 52).

4. The electrode assembly according to any one of claims 1 to 3, wherein the first or second electrode (30, 40) comprises solely one or more spirals (31) wound around an isolating core (33) being in one piece with the isolating cladding (59) of the electric connection (51) with no or one or more attachment means (37; 147).

5. The electrode assembly according to any one of claims 1 to 4, wherein attachments means (37) are provided at the first electrode (30) comprising one or more free ends (37).

6. The electrode assembly according to claim 5, wherein there are provided three or four free ends (37) spanning a triangle or square, respectively, in a plane perpendicular to the longitudinal direction of the first electrode (30) in front of the electrode end.

7. The electrode assembly according to claim 5, wherein there are provided two or three groups of one or two free ends (147) extending essentially transverse to the longitudinal direction of the first electrode (30), each group provided between a transition portion (35) at the beginning of the first electrode (30) and the tip of the electrode (30), preferably at the beginning, the tip and in the case of three groups in the middle between the beginning and the end of the electrode (30).

8. The electrode assembly according to claim 7, wherein each said group comprises two free ends (147) having an angle between 45 and 90 degree in a plane perpendicular to the longitudinal direction of the electrode (30) in front of the electrode end.

9. The electrode assembly according to any one of claims 5 to 8, wherein the free end(s) (37) are tapering each into an anchoring spike or into an anchoring hook.

## Patentansprüche

1. Implantierbare Gleichstrom-Elektrodenanordnung (20) mit zwei implantierbaren Elektroden (30, 40) und einer Steuereinheit (50), mit der die erste (30) und die zweite (40) Elektrode über Leitungen elektrisch verbunden sind, wobei die Steuereinheit (50) dazu ausgebildet ist, eine Potentialdifferenz zwischen den zwei Elektroden (30, 40) herzustellen, so dass ein Gleichstrom zwischen den zwei Elektroden (30, 40) fliessen kann (55), wobei die erste Elektrode (30) eine Spulenelektrode ist, die dazu ausgebildet ist, in der rechten Hälfte (12) des Herzens (10) vorgesehen zu sein und eine vorbestimmte Länge hat, und wobei die zweite Elektrode eine Gegenelektrode (40) ist, wobei diese Gegenelektrode eine Spulenelektrode (40) ist, die einen Elektrodenabschnitt (31) aufweist, der ausgebildet ist, um im Koronarsinus positioniert zu werden, **dadurch gekennzeichnet, dass** die Spulenelektrode (40) ausgebildet ist, um in dem Koronarsinus in der Höhe des linken Atriums (24) positioniert zu werden und dass jenseits des Elektrodenabschnitts (31) der Spulenelektrode (40) ein isolierter Verankerungsabschnitt (42) angebracht ist, der sich über den Elektrodenabschnitt (31) hinaus erstreckt und in einer Spitze (32) endet, wobei eine vorbestimmte Länge des isolierten Verankerungsabschnitts (42) der Biegung der Vena cardiaca magna in Richtung des linken Ventrikels (118) folgt.

2. Elektrodenanordnung nach Anspruch 1, wobei der isolierte Verankerungsabschnitt (42) eine vorgebogene gekrümmte Struktur (43; 44) umfasst, um gegen eine Vielzahl von Kontaktpunkten (44) innerhalb des Querschnitts der sich verjüngenden Vena cardiaca magna bis zur Spitze (32) des isolierten Verankerungsabschnitts (42) positioniert zu werden.

3. Elektrodenanordnung nach Anspruch 1 oder 2, wobei die elektrische Verbindung zwischen den elektrisch verbundenen ersten (30) und zweiten (40) Elektroden und der Steuereinheit (50) einzelne verdrahtete isolierte Leitungen (51, 52) sind.

4. Elektrodenanordnung nach irgendeinem der Ansprüche 1 bis 3, wobei die erste oder zweite Elektrode (30, 40) nur eine oder mehrere Spiralen (31) umfasst, die um einen isolierenden Kern (33) gewickelt sind, der mit der isolierenden Umhüllung (59) der elektrischen Verbindung (51) einstückig ist, ohne dass ein oder mehrere Befestigungsmittel (37; 147) vorhanden sind.

5. Elektrodenanordnung nach irgendeinem der Ansprüche 1 bis 4, wobei an der ersten Elektrode (30) Befestigungsmittel (37) vorgesehen sind, die ein oder mehrere freie Enden (37) aufweisen.

6. Elektrodenanordnung nach Anspruch 5, wobei drei oder vier freie Enden (37) vorgesehen sind, die ein Dreieck bzw. ein Quadrat in einer Ebene senkrecht zur Längsrichtung der ersten Elektrode (30) vor dem Elektrodenende aufspannen.

7. Elektrodenanordnung nach Anspruch 5, **dadurch gekennzeichnet, dass** zwei oder drei Gruppen von einem oder zwei freien Enden (147) vorgesehen sind, die sich im Wesentlichen quer zur Längsrichtung der ersten Elektrode (30) erstrecken, wobei jede Gruppe zwischen einem Übergangsbereich (35) am Anfang der ersten Elektrode (30) und der Spitze der Elektrode (30) vorgesehen ist, vorzugsweise am Anfang, an der Spitze und bei drei Gruppen in der Mitte zwischen dem Anfang und dem Ende der Elektrode (30).

8. Elektrodenanordnung nach Anspruch 7, wobei jede Gruppe zwei freie Enden (147) aufweist, die in einer Ebene senkrecht zur Längsrichtung der Elektrode (30) vor dem Elektrodenende einen Winkel zwischen 45 und 90 Grad aufweisen.

9. Elektrodenanordnung nach irgendeinem der Ansprüche 5 bis 8, wobei das/die freie(n) Ende(n) (37) jeweils in einem Verankerungsdorn oder in einem Verankerungshaken auslaufen.

## Revendications

1. Ensemble d'électrodes implantables à courant continu (20) comprenant deux électrodes implantables (30, 40) et une unité de commande (50), à laquelle la première (30) et la seconde (40) électrodes sont électriquement connectées par des fils, l'unité de commande (50) étant configurée pour établir une différence de potentiel entre les deux électrodes (30, 40), de sorte qu'un courant continu puisse circuler (55) entre les deux électrodes (30, 40), la première électrode (30) étant une électrode hélicoïdale configurée pour être placée dans la moitié droite (12) du coeur (10) et ayant une longueur prédéterminée, et la seconde électrode étant une contre-électrode (40), cette contre-électrode étant une électrode hélicoïdale (40) ayant une partie d'électrode (31) configurée pour être positionnée dans le sinus coronaire, **caractérisée en ce que** l'électrode hélicoïdale (40) est configurée pour être positionnée dans le sinus coronaire à la hauteur de l'oreillette gauche (24) et **en ce qu'**au-delà de la partie d'électrode (31) de l'électrode hélicoïdale (40) est fixée une partie d'ancrage isolée (42) s'étendant au-delà de la partie électrode (31) se terminant par une pointe (32) avec une longueur prédéterminée de la partie d'ancrage isolée (42) poursuivre la courbure de la vena cardiaca magna en direction du ventricule gauche (118).

2. Ensemble d'électrodes selon la revendication 1, dans lequel la partie d'ancrage isolée (42) comprend une structure courbée pré-courbée (43 ; 44) à positionner contre une pluralité de points de contact (44) à l'intérieur de la section transversale de la vena cardiaca magna effilée jusqu'à l'extrémité (32) de la partie d'ancrage isolée (42).

3. Ensemble d'électrodes selon la revendication 1 ou 2, dans lequel la connexion électrique entre la première (30) et la deuxième (40) électrodes connectées électriquement et l'unité de commande (50) sont des lignes isolées à câblage unique (51, 52).

4. Ensemble d'électrodes selon l'une quelconque des revendications 1 à 3, dans lequel la première ou la deuxième électrode (30, 40) comprend uniquement une ou plusieurs spirales (31) enroulées autour d'un noyau isolant (33) d'un seul tenant avec la gaine isolante (59) de la connexion électrique (51) sans aucun ou un ou plusieurs moyens de fixation (37 ; 147).

5. Ensemble d'électrodes selon l'une quelconque des revendications 1 à 4, dans lequel les moyens de fixation (37) sont fournis à la première électrode (30) comprenant une ou plusieurs extrémités libres (37).

6. Ensemble d'électrodes selon la revendication 5, dans lequel il y a trois ou quatre extrémités libres (37) couvrant un triangle ou un carré, respectivement, dans un plan perpendiculaire à la direction longitudinale de la première électrode (30) en face de l'extrémité de l'électrode.

7. Ensemble d'électrodes selon la revendication 5, dans lequel il est prévu deux ou trois groupes d'une ou deux extrémités libres (147) s'étendant essentiellement transversalement à la direction longitudinale de la première électrode (30), chaque groupe étant prévu entre une partie de transition (35) au début de la première électrode (30) et la pointe de l'électrode (30), de préférence au début, à la pointe et, dans le cas de trois groupes, au milieu entre le début et la fin de l'électrode (30).

8. Ensemble d'électrodes selon la revendication 7, dans lequel chaque groupe comprend deux extrémités libres (147) ayant un angle entre 45 et 90 degrés dans un plan perpendiculaire à la direction longitudinale de l'électrode (30) en avant de l'extrémité de l'électrode.

9. Ensemble d'électrodes selon l'une quelconque des revendications 5 à 8, dans lequel la ou les extrémités libres (37) se rétrécissent chacune en une pointe d'ancrage ou en un crochet d'ancrage.
